# DEMANDE DE BREVET EUROPEEN

(11) **EP 3 881 701 A1**
(43) Date de publication de la demande: **22.09.2021**
(21) Numéro de dépôt: 21162259.2
(22) Date de dépôt: 12.03.2021
(51) Int. Cl.: A41D 1/00, B60P 3/22

(54) **DISPOSITIF DE SURVEILLANCE SUR SITE D'UN HYDROCUREUR ET D'UN OPÉRATEUR DE L HYDROCUREUR, ET SYSTÈME DE SURVEILLANCE GLOBALE**

(30) Priorité: 20.03.2020 FR 2002764
(71) Demandeur: ORTEC EXPANSION, 13799 Aix En Provence Cedex 3 (FR)
(72) Inventeur: RIVARD, Daniel, 75007 Paris (FR)
(74) Mandataire: Gevers & Orès

(57) **Abrégé**

- Dispositif de surveillance sur site d'un hydrocureur et d'un opérateur de l'hydrocureur, et système de surveillance globale.
- Le dispositif (1) de surveillance sur site comporte une unité de surveillance (11) comprenant au moins un habit destiné à être porté par l'opérateur, qui est pourvu de capteurs (CA1 à CAn, CB1 à CBm) pour mesurer des valeurs de paramètres liés audit opérateur et/ou à son environnement et une liaison de transmission pour communiquer avec une unité de surveillance locale (4) montée sur l'hydrocureur, ladite unité de surveillance locale (4) étant apte à communiquer avec une unité de contrôle (31) d'un poste de contrôle (29) situé à distance du site de travail de manière à permettre de réaliser une surveillance à distance de l'opérateur et de l'hydrocureur.

## Description

### DOMAINE TECHNIQUE

La présente invention concerne une unité de surveillance pour un opérateur d'un hydrocureur, un dispositif de surveillance sur site comprenant un hydrocureur et ladite unité de surveillance, ainsi qu'un système de surveillance globale (avec un contrôle et une intervention à distance).

### ÉTAT DE LA TECHNIQUE

On sait qu'un hydrocureur de type industriel est destiné à des travaux de nettoyage d'équipements et d'installations industrielles. On utilise notamment un hydrocureur lors des travaux de pompage et de nettoyage sur les sites pétroliers et parapétroliers.

On sait qu'un hydrocureur utilisé dans des complexes pétroliers et parapétroliers a pour objet de :
- pomper des produits pétroliers ; et
- d'effectuer divers travaux (de décapage, de débouchage, de remise à niveau de tubes d'échangeurs, de nettoyage d'enceintes ou de bacs, ...) à haute pression.

Ces travaux peuvent être dangereux pour l'opérateur.

En particulier, dans le cas du pompage, peuvent exister :
- un risque physique, par exemple la chute brutale de l'opérateur sur le site, un risque d'atmosphère dangereuse, un risque cardiaque ou respiratoire ; et
- des risques matériels dus à un fonctionnement anormal de l'hydrocureur tel qu'une montée anormale en température du moteur du porteur ou d'un système de pompage de l'hydrocureur, une atmosphère dans l'environnement de l'hydrocureur incompatible avec la sécurité de fonctionnement, une perte d'équipotentialité d'une ligne d'aspiration.

En outre, dans le cas de travaux à haute pression, il existe des risques physiques pour l'opérateur, dus par exemple à un éclatement d'un flexible ou à un disfonctionnement d'un outil.

Il existe donc un besoin de moyens permettant de surveiller un opérateur d'un hydrocureur et/ou des équipements de l'hydrocureur pour pouvoir détecter un problème ou un danger, ou un risque de problème ou de danger et ainsi pouvoir intervenir à partir d'un poste à distance, sur l'hydrocureur ou venir au secours de l'opérateur.

### EXPOSÉ DE L'INVENTION

La présente invention a pour objet de répondre à ce besoin. Elle concerne tout d'abord une unité de surveillance pour un opérateur d'un hydrocureur.

Selon l'invention, ladite unité de surveillance comporte :
- au moins un habit destiné à être porté par l'opérateur, ledit habit étant pourvu d'un premier ensemble de capteurs configurés pour mesurer des valeurs de paramètres liés audit opérateur et/ou à son environnement ; et
- au moins un émetteur radioélectrique configuré pour émettre sous forme d'ondes radioélectriques de premières informations, et au moins des valeurs mesurées par au moins certains desdits capteurs dudit premier ensemble de capteurs.

Ainsi, grâce aux capteurs, on est en mesure de surveiller (en temps réel) l'opérateur et son environnement, et grâce à l'émetteur radioélectrique, on peut transmettre les valeurs mesurées à un poste de contrôle pour une surveillance à distance comme précisé ci-dessous.

Avantageusement, ledit habit correspond à l'un des éléments suivants : une combinaison de travail, un sous-vêtement.

Par ailleurs, de façon avantageuse, ledit premier ensemble de capteurs comporte des capteurs aptes à mesurer au moins certains des paramètres suivants de l'opérateur :
- une ou plusieurs données cardiaques (rythme cardiaque, tension, ...) ;
- une ou plusieurs données respiratoires ;
- sa température corporelle ;
- son pouls ;
- sa position.

En outre, avantageusement, ledit premier ensemble de capteurs comporte des capteurs aptes à mesurer au moins certains des paramètres suivants de l'environnement de l'opérateur :
- la présence d'au moins un gaz ;
- la température ;
- le vent.

La présente invention concerne également un dispositif de surveillance sur site.

Selon l'invention, ledit dispositif de surveillance sur site comporte au moins une unité de surveillance telle que celle décrite ci-dessus et une unité de surveillance locale destinée à être montée sur un hydrocureur. Cette unité de surveillance locale comporte au moins un récepteur radioélectrique configuré pour pouvoir recevoir les premières informations émises sous forme d'ondes radioélectriques par l'émetteur radioélectrique de ladite unité de surveillance et un émetteur radioélectrique apte à émettre sous forme d'ondes radioélectriques de secondes informations (à destination d'un poste de contrôle situé à distance).

Avantageusement, ladite unité de surveillance locale comporte un second ensemble de capteurs destinés à être montés sur l'hydrocureur, ledit second ensemble de capteurs comportant des capteurs configurés pour mesurer des valeurs de paramètres liés audit hydrocureur et/ou à son environnement.

En outre, de façon avantageuse, ledit second ensemble de capteurs comporte des capteurs aptes à mesurer au moins certains des paramètres suivants de l'hydrocureur :
- des paramètres d'un moteur de l'hydrocureur ;
- des paramètres d'un équipement de l'hydrocureur.

De plus, avantageusement, ledit second ensemble de capteurs comporte des capteurs aptes à mesurer au moins certains des paramètres suivants de l'environnement de l'hydrocureur :
- la présence d'au moins un gaz ;
- la température ;
- le vent.

Par ailleurs, ledit dispositif de surveillance sur site comporte :
- au moins un élément d'alerte visuel et/ou sonore ; et/ou
- une unité centrale pour le traitement de valeurs mesurées ; et/ou
- l'hydrocureur sur lequel est montée ladite unité de surveillance locale.

La présente invention concerne également un système de surveillance globale, permettant un contrôle et une intervention à distance.

Selon l'invention, ledit système de surveillance globale comporte au moins un dispositif de surveillance sur site tel que celui décrit ci-dessus, et une unité de contrôle à distance. Cette unité de contrôle à distance comprend au moins une seconde unité de transmission radioélectrique apte au moins à recevoir des secondes informations émises par une première unité de transmission radioélectrique de ladite unité de surveillance locale.

Avantageusement, l'unité de contrôle à distance comporte au moins l'un des éléments suivants :
- au moins un élément d'alerte visuel et/ou sonore ;
- au moins un élément d'affichage ;
- au moins un élément de traitement de données.

Par ailleurs, de façon avantageuse, l'unité de contrôle à distance comporte une unité de commande configurée pour générer de ordres de commande permettant de commander à distance au moins un équipement de l'hydrocureur, lesdits ordres de commande étant émis par ladite seconde unité de transmission radioélectrique et reçus par ladite première unité de transmission radioélectrique.

En outre, avantageusement, l'unité de contrôle à distance comporte une unité d'alerte configurée pour générer de ordres de déclenchement d'alerte permettant de commander à distance au moins un élément d'alerte du dispositif de surveillance sur site, lesdits ordres de déclenchement d'alerte étant émis par ladite seconde unité de transmission radioélectrique et reçus par ladite première unité de transmission radioélectrique.

### BRÈVE DESCRIPTION DES FIGURES

Les figures annexées feront bien comprendre comment l'invention peut être réalisée. Sur ces figures, des références identiques désignent des éléments semblables.
La figure 1 est schéma synoptique d'un mode de réalisation particulier d'un système de surveillance globale.
La figure 2 est une vue schématique d'un exemple particulier d'un hydrocureur et d'un opérateur réalisant un pompage.
La figure 3 est une vue schématique d'une combinaison d'un opérateur, pourvue de capteurs.
La figure 4 est une vue schématique d'un sous-vêtement d'un opérateur, pourvu de capteurs.
La figure 5 est une vue schématique montrant un opérateur utilisant une lance à haute pression.
La figure 6 est une vue schématique d'un poste de contrôle (et d'intervention) situé à distance.

### DESCRIPTION DÉTAILLÉE

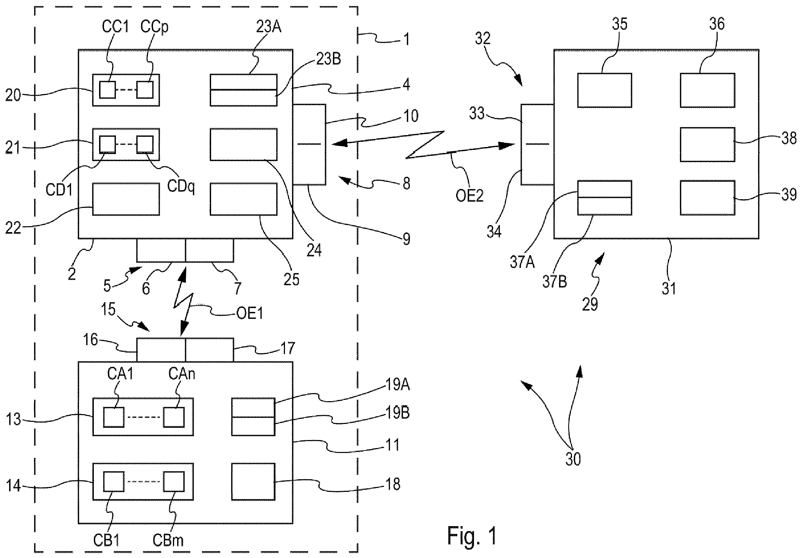

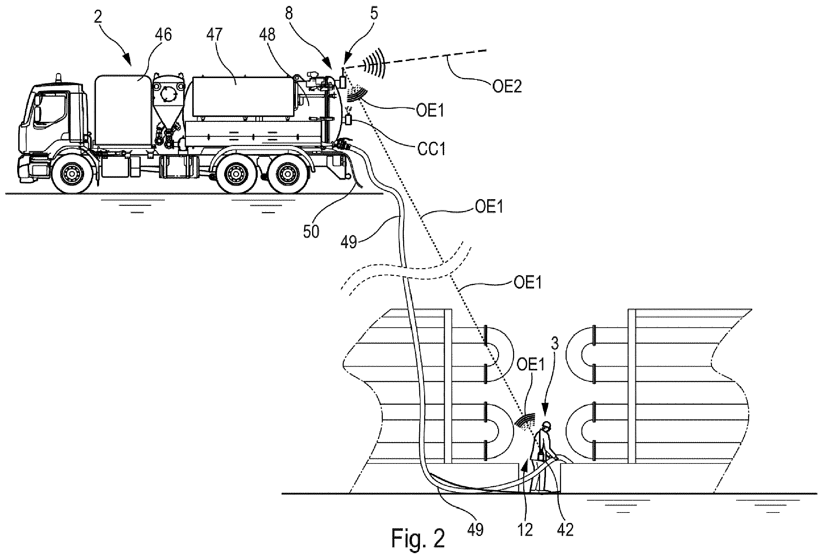

Le dispositif 1, illustrant l'invention et représenté schématiquement sur la figure 1, est un dispositif de surveillance sur site, destiné à la surveillance d'un site d'opération d'un hydrocureur 2 (figure 2).

Plus précisément, le dispositif 1 est destiné à la surveillance d'au moins certains des éléments suivants du site d'opération : l'hydrocureur 2, l'environnement de l'hydrocureur 2, au moins un opérateur 3 (figure 2) de l'hydrocureur 2 et l'environnement de l'opérateur 3 (notamment si l'opérateur 3 est éloigné de l'hydrocureur 2).

Ledit dispositif 1 (de surveillance sur site) comporte une unité de surveillance locale (ci-après « unité 4 ») qui est montée sur l'hydrocureur 2.

L'unité 4 (de surveillance locale) comporte une unité de transmission radioélectrique 5 comprenant au moins un récepteur radioélectrique 6. L'unité de transmission radioélectrique 5 est destinée à une communication avec l'opérateur comme précisé ci-dessous. Le récepteur radioélectrique 6 est configuré pour pouvoir recevoir des informations émises sous forme d'ondes radioélectriques OE1 à basse fréquence.

Dans un premier mode de réalisation, l'unité de transmission radioélectrique 5 comprend uniquement le récepteur radioélectrique 6.

Dans un second mode de réalisation, l'unité de transmission radioélectrique 5 comprend, en plus du récepteur radioélectrique 6, un émetteur radioélectrique 7. L'émetteur radioélectrique 7 est configuré pour pouvoir émettre des informations émises sous forme d'ondes radioélectriques OE1.

En outre, l'unité 4 (de surveillance locale) comporte une unité de transmission radioélectrique 8 comprenant au moins émetteur radioélectrique 9. L'unité de transmission radioélectrique 8 est destinée à une communication avec un poste de contrôle 29 à distance comme précisé ci-dessous. L'émetteur radioélectrique 9 est configuré pour pouvoir émettre des informations sous forme d'ondes radioélectriques OE2.

Dans un premier mode de réalisation, l'unité de transmission radioélectrique 8 comprend uniquement l'émetteur radioélectrique 9.

Dans un second mode de réalisation, l'unité de transmission radioélectrique 8 comprend, en plus de l'émetteur radioélectrique 9, un récepteur radioélectrique 10. Le récepteur radioélectrique 10 est configuré pour pouvoir recevoir des informations émises sous forme d'ondes radioélectriques OE2.

Dans une variante de réalisation (non représentée), le dispositif 1 comporte, au lieu des deux unités de transmission radioélectrique 5 et 8, une seule unité de transmission radioélectrique pour une communication, à la fois avec le site de travail de l'opérateur 3 et avec le poste de contrôle 29 à distance.

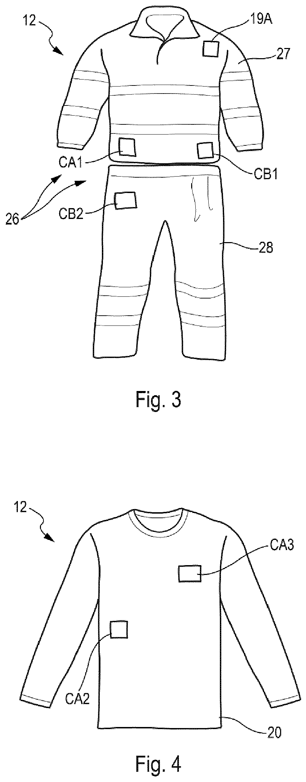

Par ailleurs, l'opérateur 3 est muni d'une unité de surveillance 11 (personnelle) qui est intégrée, de préférence, au moins en partie dans au moins un habit 12 (figures 3 et 4) porté par l'opérateur 3.

L'unité de surveillance 11, qui est destinée à la surveillance de l'opérateur et de son environnement de travail, est pourvue d'un ensemble 13 de capteurs. Cet ensemble 13 comporte, comme représenté sur la figure 1, une pluralité de capteurs CA1 à CAn, n'étant un entier. Ces capteurs CA1 à CAn sont configurés pour mesurer des valeurs de paramètres liés directement à la personne qu'est l'opérateur.

Plus précisément, ledit ensemble 13 peut comporter, parmi les capteurs CA1 à CAn :
- un ou plusieurs capteurs pour mesurer une ou plusieurs données cardiaques (rythme cardiaque, tension, ...) de l'opérateur 3, en particulier pour pouvoir détecter un accident cardiaque de l'opérateur ou un risque imminent d'accident cardiaque ;
- un ou plusieurs capteurs pour mesurer une ou plusieurs données respiratoires de l'opérateur 3, en particulier pour pouvoir détecter une insuffisance respiratoire ;
- un capteur thermique, en particulier un thermomètre, pour mesurer la température corporelle de l'opérateur 3 et pouvoir détecter de la fièvre ;
- un capteur pour mesurer le pouls de l'opérateur 3, par exemple pour pouvoir détecter un rythme cardiaque trop élevé ;
- un ou plusieurs capteurs, par exemple des gyromètres et/ou des accéléromètres, pour mesurer la position de l'opérateur, et éventuellement certains de ses mouvements. Ces capteurs peuvent, notamment, être configurés pour mesurer des données qui sont représentatives d'une chute de l'opérateur 3.

L'unité de surveillance 11, qui est destinée à la surveillance de l'opérateur et de son environnement de travail, est également pourvue d'un ensemble 14 de capteurs. Cet ensemble 14 comporte, comme représenté sur la figure 1, une pluralité de capteurs CB1 à CBm, m étant un entier. Ces capteurs CB1 à CBm sont configurés pour mesurer des valeurs de paramètres liés à l'environnement de travail de l'opérateur, afin de pouvoir évaluer notamment la qualité de l'atmosphère environnante.

Plus précisément, ledit ensemble 13 peut comporter, parmi les capteurs CB1 à CBm :
- un ou plusieurs capteurs pour mesurer, dans l'environnement de travail de l'opérateur 3, la présence d'un ou de plusieurs gaz, potentiellement nocifs ; et/ou
- un capteur thermique, en particulier un thermomètre, pour mesurer la température de l'environnement de travail de l'opérateur 3 et détecter par exemple une température anormalement élevée ; et/ou
- un capteur pour mesurer le vent, dans l'environnement de travail de l'opérateur 3, notamment en cas de travail à l'extérieur.

L'unité de surveillance 11 comporte également une unité de transmission radioélectrique 15 comprenant au moins émetteur radioélectrique 16. L'unité de transmission radioélectrique 15 est destinée à une communication avec l'unité 4 de l'hydrocureur 2, comme précisé ci-dessous. L'émetteur radioélectrique 16 est configuré pour pouvoir émettre des informations émises sous forme d'ondes radioélectriques OE1 qui sont reçues par le récepteur radioélectrique 6 de l'unité de surveillance 4 de l'hydrocureur 2.

Dans un premier mode de réalisation simplifié, l'émetteur radioélectrique 16 envoie, comme informations, directement les valeurs mesurées par au moins certains des capteurs des ensembles 13 et 14.

Ces valeurs peuvent être communiquées, en continu ou périodiquement, notamment en fonction de leur importance dans la surveillance.

Dans un deuxième mode de réalisation, l'unité de surveillance 11 comporte un élément de traitement 18 qui est relié à au moins certains des capteurs des ensembles 13 et 14 et qui réalise un prétraitement de certaines valeurs mesurées, par exemple une comparaison d'une valeur mesurée à une valeur de seuil pour pouvoir détecter une anomalie. Dans ce second mode de réalisation, l'émetteur radioélectrique 16 envoie des informations à destination de l'hydrocureur 2 uniquement en cas de détection d'une anomalie par l'élément de traitement 18 pour informer de cette anomalie.

En outre, un troisième mode de réalisation correspond à la combinaison des premier et deuxième modes de réalisation précédents.

Par ailleurs, dans un premier mode de réalisation, l'unité de transmission radioélectrique 15 comprend uniquement l'émetteur radioélectrique 16.

En outre, dans un second mode de réalisation, l'unité de transmission radioélectrique 15 comprend, en plus de l'émetteur radioélectrique 16, un récepteur radioélectrique 17. Le récepteur radioélectrique 17 est configuré pour pouvoir recevoir des informations émises sous forme d'ondes radioélectriques OE1 par l'émetteur radioélectrique 7 de l'unité 4 de l'hydrocureur, comme précisé ci-dessous.

On prévoit des moyens pour sécuriser la transmission entre l'unité de transmission radioélectrique 15 et l'unité de transmission radioélectrique 8.

L'unité de surveillance 11 comporte au moins un élément d'alerte (ou avertisseur) 19A, 19B. Il peut s'agir d'un élément d'alerte visuel 19A qui est de préférence porté par l'opérateur 3 et qui est par exemple fixé sur la veste 27 d'une combinaison de travail 26 (figure 3). Cet élément d'alerte visuel 19A est apte à émettre un signal visuel d'alerte tel qu'une lumière clignotante. Il peut également s'agir d'un élément d'alerte sonore 19B, tel qu'une sirène, qui est de préférence porté par l'opérateur et qui est apte à émettre un signal sonore d'alerte.

Le ou les éléments d'alerte 19A et 19B peuvent être déclenchés de différentes manières selon le mode de réalisation envisagé. En particulier :
- le ou les éléments d'alerte 19A et 19B peuvent être reliés directement à au moins certains des capteurs des ensembles 13 et 14 et être déclenchés dès qu'une valeur particulière est mesurée par l'un des capteurs, notamment quand une valeur seuil est dépassée. Ceci peut, notamment, être le cas lorsqu'un capteur détecte un gaz nocifs ; et/ou
- le ou les éléments d'alerte 9A et 9B peuvent être liés à l'élément de traitement 18 et être déclenchés dès qu'une anomalie est détectée par l'élément de traitement 18, par exemple lorsque le rythme cardiaque de l'opérateur dépasse une certaine valeur ; et/ou
- le ou les éléments d'alerte 9A et 9B peuvent être déclenchés sur un ordre émis via l'émetteur radioélectrique 7 de l'hydrocureur 2 et reçu par le récepteur radioélectrique 17. Comme précisé ci-dessous, un tel ordre peut être généré par un poste de contrôle 29 à distance.

Au moins une partie de l'unité de surveillance 11 est intégrée dans un ou plusieurs habits 12 portés par l'opérateur 3 lors de son travail.

Cet habit 12 peut correspondre à une combinaison de travail 26, comme représenté sur la figure 3. A titre d'illustration, dans l'exemple de la figure 3, la combinaison de travail 26 comprenant une veste 27 et un pantalon 28, est pourvue de capteurs CA1, CB1 et CB2 agencés à différents endroits de ladite combinaison de travail 26.

Cet habit 12 peut également correspondre à un sous-vêtement 20, comme représenté sur la figure 4. A titre d'illustration, dans l'exemple de la figure 4, le sous-vêtement 20 est pourvu de capteurs CA2 et CA3 agencés à différents endroits dudit sous-vêtement 20.

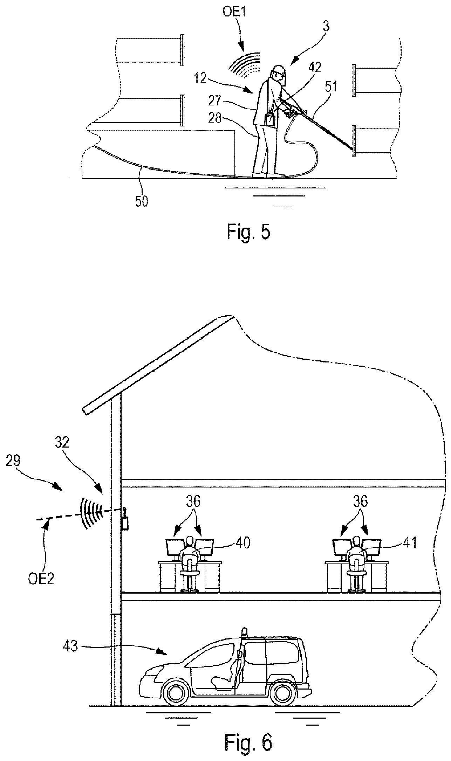

Certains des éléments de l'unité de surveillance 11, par exemple un élément d'alerte, peuvent également être intégrés dans un élément portatif 42 (figures 2 et 5), tel qu'une sacoche ou un autre élément portatif, que porte l'opérateur 3 ou qui est posé à proximité de lui lorsqu'il travaille.

Par ailleurs, l'unité 4 (de surveillance locale) qui est également destinée à la surveillance de l'hydrocureur 2 et de son environnement, est pourvue d'un ensemble 20 de capteurs. Cet ensemble 20 comporte, comme représenté sur la figure 1, une pluralité de capteurs CC1 à CCp, p étant un entier. Ces capteurs CC1 à CCp sont configurés pour mesurer des valeurs de paramètres liés audit hydrocureur 2. Il peut notamment s'agir :
- de paramètres d'un moteur de l'hydrocureur 2 ; et
- de paramètres d'un équipement, par exemple d'un système de pompage, de l'hydrocureur 2.

Plus précisément, ledit ensemble 20 peut comporter, parmi les capteurs CC1 à CCp, un ou plusieurs capteurs pour mesurer un ou plusieurs des paramètres suivants du moteur de l'hydrocureur 2 :
- une montée anormale en température du moteur ; et/ou
- une perte de la pression d'huile du moteur ; et/ou
- un régime anormal du moteur.

Ledit ensemble 20 peut également comporter, parmi les capteurs CC1 à CCp, un ou plusieurs capteurs pour mesurer un ou plusieurs des paramètres suivants d'un équipement de l'hydrocureur :
- concernant un système de pompage 46 (figure 2) : une montée anormale en température du moteur du système de pompage 46, une perte de la pression d'huile de l'entraînement du système de pompage 46, un régime anormal du système de pompage 46 ; et/ou
- une défaillance du niveau de remplissage d'une cuve de collecte 48 (figure 2) de l'hydrocureur 3 ; et/ou
- une perte de l'équipotentialité d'une ligne d'aspiration.

L'unité 4 est également pourvue d'un ensemble 21 de capteurs. Cet ensemble 21 comporte, comme représenté sur la figure 1, une pluralité de capteurs CD1 à CDq, q étant un entier. Ces capteurs CD1 à CDq sont configurés pour mesurer des valeurs de paramètres liés à l'environnement de l'hydrocureur 2.

Plus précisément, ledit ensemble 21 peut comporter, parmi les capteurs CD1 à CDq :
- un ou plusieurs capteurs pour mesurer, dans l'environnement de l'hydrocureur 2, la présence d'un ou de plusieurs gaz, potentiellement nocifs ; et/ou
- un capteur thermique, en particulier un thermomètre, pour mesurer la température de l'environnement de l'hydrocureur 2 et pouvoir détecter une température anormalement élevée ; et/ou
- un capteur pour mesurer le vent de l'environnement de l'hydrocureur 2.

Par ailleurs l'unité 4 comporte également une unité centrale 22 qui est reliée à au moins certains des capteurs des ensembles 20 et 21 et qui réalise un traitement de certaines valeurs mesurées, par exemple une comparaison d'une valeur mesurée à une valeur de seuil pour détecter une anomalie.

L'unité centrale 22 peut également réaliser un traitement de valeurs mesurées par des capteurs des ensembles 13 et 14 de l'opérateur, valeurs qui sont reçues par le récepteur radioélectrique 7.

L'unité 4 comporte également au moins un élément d'alerte 23A, 23B. Il peut s'agir d'un élément d'alerte visuel 23A qui est, de préférence, monté sur l'hydrocureur 2 et qui est apte à émettre un signal visuel d'alerte, tel qu'une lumière clignotante. Il peut également s'agir d'un élément d'alerte sonore 23B, tel qu'une sirène, qui est de préférence monté sur l'hydrocureur 2 et qui est apte à émettre un signal sonore d'alerte.

Le ou les éléments d'alerte 23A et 23B peuvent être déclenchés de différentes manières selon le mode de réalisation envisagé. En particulier :
- le ou les éléments d'alerte 23A et 23B peuvent être liés à au moins certains des capteurs des ensembles 20 et 21 et être déclenchés dès qu'une valeur particulière est mesurée par l'un de ces capteurs ; et/ou
- le ou les éléments d'alerte 23A et 23B peuvent être liés à l'unité centrale 22 et être déclenchés dès qu'une anomalie est détectée par l'unité centrale 22 ; et/ou
- le ou les éléments d'alerte 23A et 23B peuvent être déclenchés sur un ordre reçu par le récepteur radioélectrique 10, à partir d'un poste de contrôle 29 situé à distance, comme précisé ci-dessous.

Le dispositif 1 (de surveillance sur site) fait partie d'un système de contrôle à distance et éventuellement d'intervention (ci-après « système 30 »), comme représenté sur la figure 1.

Ce système 30 (qui est un système de surveillance globale) comporte, en plus dudit dispositif 1 (de surveillance sur site tel que décrit ci-dessus), une unité de contrôle à distance 31 agencée au niveau du poste de contrôle 29 situé à distance, représenté sur la figure 6.

L'unité de contrôle à distance 31 est pourvue d'une unité de transmission radioélectrique 32.

L'unité de transmission radioélectrique 32 comprend, comme représenté sur la figure 1, au moins récepteur radioélectrique 33. L'unité de transmission radioélectrique 32 est destinée à une communication avec l'unité 4 comme précisé ci-dessous. Le récepteur radioélectrique 33 est configuré pour pouvoir recevoir des informations émises sous forme d'ondes radioélectriques OE2 par l'émetteur radioélectrique 9 de l'unité 4 (de surveillance locale) de l'hydrocureur 3. On prévoit des moyens pour sécuriser la transmission entre l'unité de transmission radioélectrique 32 et l'unité de transmission radioélectrique 8.

Dans un premier mode de réalisation, l'unité de transmission radioélectrique 32 comprend uniquement le récepteur radioélectrique 33.

Dans un second mode de réalisation, l'unité de transmission radioélectrique 32 comprend, en plus du récepteur radioélectrique 33, un émetteur radioélectrique 34. L'émetteur radioélectrique 34 est configuré pour pouvoir émettre des informations (et notamment des ordres, de commande ou d'alerte) sous forme d'ondes radioélectriques OE2 à destination de l'hydrocureur 2, en particulier pour déclencher un élément d'alerte ou pour prendre en main un équipement de l'hydrocureur comme précisé ci-dessous.

La transmission entre, d'une part, l'unité de transmission radioélectrique 8 de l'hydrocureur 3 et, d'autre part, l'unité de transmission radioélectrique 32 du poste de contrôle 29, est sécurisée.

L'unité de contrôle à distance 31 comporte un ensemble de moyens permettant d'informer et d'avertir d'une anomalie des personnes 40 et 41 (figure 6) installées (à distance du site de travail) au poste de contrôle 29. Ces personnes 30 et 31 sont employées à la surveillance à distance à la fois de l'opérateur, de l'hydrocureur et du site de travail, et à intervenir à distance si nécessaire. De préférence, il s'agit au moins d'un technicien 40 et d'un médecin 41 qui sont présents au poste de contrôle 29 au moins pendant la durée de travail de l'opérateur 3.

L'unité de contrôle à distance 31 comporte un élément de traitement de données 35 configuré pour réaliser un traitement de certaines valeurs mesurées qui sont reçues par le récepteur radioélectrique 34, par exemple une comparaison d'une valeur mesurée à une valeur de seuil pour détecter une anomalie.

L'unité de contrôle à distance 31 comporte également au moins un élément d'affichage 36, tel qu'un écran, qui permet d'afficher des valeurs brutes mesurées et/ou des valeurs traitées par l'unité centrale 22 ou par l'élément de traitement de données 35.

L'unité de contrôle à distance 31 comporte également au moins un élément d'alerte 37A, 37B. Il peut s'agir d'un élément d'alerte visuel 37A qui est installé dans le poste de contrôle 29 et qui est apte à émettre un signal visuel d'alerte tel qu'une lumière clignotante. Il peut également s'agir d'un élément d'alerte sonore 37B, tel qu'une sirène, qui est également installé dans le poste de contrôle 29 et qui est apte à émettre un signal sonore d'alerte.

Le ou les éléments d'alerte 37A et 37B peuvent être déclenchés de différentes manières selon le mode de réalisation envisagé. En particulier :
- le ou les éléments d'alerte 37A et 37B peuvent être déclenchés dès qu'une valeur particulière est mesurée par l'un des capteurs des ensembles 13, 14, 20 et 21 ; et/ou
- le ou les éléments d'alerte 37A et 37B peuvent être déclenchés dès qu'une anomalie est détectée par l'unité centrale 22 ou par l'élément de traitement de données 35.

Par ailleurs, dans un mode de réalisation particulier, l'unité de contrôle à distance 31 comporte une unité de commande 38. Cette unité de commande 38 est configurée pour permettre à une personne du poste de contrôle 29, notamment le technicien 40, de générer des ordres de commande. Ces ordres de commande sont destinés à commander à distance au moins un équipement de l'hydrocureur, par exemple l'arrêter en cas de dysfonctionnement ou de danger.

Les ordres de commande sont émis par l'unité de transmission radioélectrique 32 du poste de contrôle 29 et reçus par l'unité de transmission radioélectrique 8 du dispositif 1 qui les transmet à un élément de commande 24 de l'hydrocureur 3 ou à un élément de commande 25 d'un équipement de l'hydrocureur 3.

En outre, dans un mode de réalisation particulier, l'unité de contrôle à distance 31 comporte une unité d'alerte 39. Cette unité d'alerte 39 est configurée pour permettre à une personne du poste de contrôle 29, notamment le technicien 40, de générer de ordres de déclenchement d'alerte permettant de déclencher à distance au moins un élément d'alerte 19A, 19B, 23A, 23B du dispositif 1 (de surveillance sur site).

Ces ordres de déclenchement d'alerte sont émis par l'unité de transmission radioélectrique 32 du poste de contrôle 29 et reçus par l'unité de transmission radioélectrique 8 du dispositif 1 qui les transmet à l'élément d'alerte concerné.

Les personnes qui surveillent à distance le site de travail, notamment le technicien 40 (pour une surveillance et un soutien techniques) et le médecin 41 (pour une surveillance et un soutien médicaux), sont donc informées en temps réel de toute anomalie, danger ou accident survenant sur le site de travail et peuvent intervenir à distance.

Ils disposent de différents moyens d'intervention, qu'ils peuvent utiliser en fonction du problème détecté. En particulier, ils peuvent déclencher une alerte sur le site de travail et ils peuvent également prendre en main un équipement de l'hydrocureur, et notamment l'arrêter.

Ils disposent également d'un ou de plusieurs véhicules d'intervention 43 (figure 6) permettant à un technicien ou à un médecin de rejoindre le site de travail, s'il n'est pas trop éloigné. Ce véhicule d'intervention 43 est notamment prévu pour une assistance médicale de proximité.

Le système 30, tel que décrit ci-dessus, permet de surveiller, à partir du poste de contrôle 29, simultanément une pluralité d'hydrocureurs travaillant sur un même site ou sur différents sites éloignés les uns des autres.

Le système 30 permet de surveiller tout type de travail réalisé par l'opérateur 3 avec l'hydrocureur 2.

A titre d'illustration, l'hydrocureur 2 tel que représenté sur la figure 2, comporte un système de pompage 46, une cuve à eau 47 pour réaliser des opérations de netoyage, et une cuve de collecte 48 pour collecter des produits pompés par le système de pompage 46. Il comporte également un flexible d'aspiration 49 (pour le pompage) et un flexible haute pression 50 (pour le nettoyage). L'hydrocureur 2 comporte ainsi un ensemble d'équipements fonctionnels permettant de réaliser des opérations de pompage et de nettoyage. L'hydrocureur 20 comporte en outre les unités de transmission radioélectrique 5 et 8, des éléments d'alerte, et des capteurs des ensembles 20 et 21, dont un seul CC1 est représenté sur la figure 2.

Dans l'exemple de la figure 2, l'opérateur 3 est en train d'aspirer des produits, par exemple des produits pétroliers, via le flexible d'aspiration 49. Ces produits sont aspirés dans la cuve de collecte 48 de l'hydrocureur 2.

La figure 5 illustre un autre exemple de travail de l'opérateur 3, avec l'hydrocureur 3 de la figure 2. Dans cet exemple, l'opérateur 3 effectue des travaux de nettoyage à l'aide d'une lance à haute pression 51 alimentée par le flexible à haute pression 49 de l'hydrocureur 2.

A titre d'illustration, non limitative, on cite ci-après différentes situations particulières susceptibles de se produire, et les surveillances, actions et/ou interventions pouvant réalisées dans ces situations particulières.

Premièrement, en cas de chute de l'opérateur 3 sur le site de travail, l'opérateur 3 étant suivi en permanence grâce aux capteurs incorporés dans le ou les habits 12 qu'il porte, le poste de contrôle 29 est averti de cette chute, via la liaison à basse fréquence entre l'équipement de l'opérateur 3 et l'hydrocureur 2 qui est, lui-même, en liaison avec le poste de contrôle 29 situé à distance.

Deuxièmement, pour répondre à un risque d'atmosphère dangereuse, au moins un capteur porté par l'opérateur 3 et au moins un capteur installé sur l'hydrocureur 2, informent le poste de contrôle 29, ce qui permet, instantanément, si nécessaire, une prise en main tant au niveau médical qu'au niveau matériel.

Troisièmement, en cas de fonctionnement anormal au niveau de l'hydrocureur 2 (montée anormale en température du moteur de l'hydrocureur 2 ou d'un système de pompage 46, perte de pression d'huile du moteur de l'hydrocureur 2 ou de l'entraînement du système de pompage 46, régime anormal du moteur de l'hydrocureur 2 ou du système de pompage 46, défaillance du niveau de remplissage de la cuve de collecte 48, perte de l'équipotentialité de la ligne d'aspiration), le technicien 40 du poste de contrôle 29 est informé, et il réalise les actions appropriées telles qu'une prise en main à distance, et notamment un arrêt de l'équipement défaillant.

Quatrièmement, en cas d'éclatement d'un flexible ou d'un outil, le poste de contrôle 29 est informé grâce à la surveillance de l'ensemble de la ligne. Si une rupture brutale de la pression est détectée alors que la pompe est en débit, cette dernière est automatiquement stoppée. L'état (médical) de l'opérateur est également surveillé par le poste de contrôle 29.

Cinquièmement, lorsqu'un opérateur blessé par des travaux, le suivi permanent de l'opérateur 3, notamment au niveau cardiaque, signale aussitôt l'accident, et l'installation est mise à l'arrêt. Dans le même temps, le médecin 41 dans le poste de contrôle 29 prend en main la situation médicale de l'opérateur 3.

Par conséquent, dans le poste de contrôle 29, à chaque alerte, un technicien 40 et un médecin 41 apportent le soutien technique approprié, voire la prise en main de l'équipement, et la bonne réponse médicale avec, si nécessaire, l'envoi sur le site de travail d'une équipe de secouristes.

## Revendications

1. Dispositif de surveillance sur site comportant un hydrocureur (2), au moins une unité de surveillance (11) pour un opérateur de l'hydrocureur (2) et une unité de surveillance locale (4) montée sur l'hydrocureur (2), l'hydrocureur (2) étant destiné à des travaux de nettoyage d'équipements et d'installations industrielles, l'unité de surveillance (11) comportant :
- au moins un habit (12) destiné à être porté par l'opérateur (3), ledit habit (12) étant pourvu d'au moins un premier ensemble (13, 14) de capteurs (CA1 à CAn, CB1 à CBm) configurés pour mesurer des valeurs de paramètres liés audit opérateur et/ou à son environnement ; et
- au moins un émetteur radioélectrique (16) configuré pour émettre sous forme d'ondes radioélectriques (OE1) de premières informations, et au moins des valeurs mesurées par au moins certains desdits capteurs (CA1 à CAn, CB1 à CBm) dudit premier ensemble (13, 14) de capteurs (CA1 à CAn, CB1 à CBm),
ladite unité de surveillance locale (4) comportant au moins un récepteur radioélectrique (6) configuré pour pouvoir recevoir les premières informations émises sous forme d'ondes radioélectriques (OE1) par l'émetteur radioélectrique (16) de l'unité de surveillance (11) et une unité de transmission radioélectrique (8) destinée à une communication avec un poste de contrôle (29) à distance, via une transmission sécurisée.

2. Dispositif selon la revendication 1,
**caractérisé en ce que** ledit habit (12) correspond à l'un des éléments suivants : une combinaison de travail (26), un sous-vêtement (20).

3. Dispositif selon l'une des revendications 1 et 2,
**caractérisé en ce que** ledit premier ensemble (13) de capteurs comporte des capteurs (CA1 à CAn) aptes à mesurer au moins certains des paramètres suivants de l'opérateur (3) :
- une ou plusieurs données cardiaques ;
- une ou plusieurs données respiratoires ;
- sa température corporelle ;
- son pouls ;
- sa position.

4. Dispositif selon l'une quelconque des revendications 1 à 3, **caractérisé en ce que** ledit premier ensemble (14) de capteurs comporte des capteurs (CB1 à CBm) aptes à mesurer au moins certains des paramètres suivants de l'environnement de l'opérateur (3) :
- la présence d'au moins un gaz ;
- la température ;
- le vent.

5. Dispositif selon l'une quelconque des revendications précédentes, **caractérisé en ce que** ladite unité de surveillance locale (4) comporte au moins un second ensemble (20, 21) de capteurs (CC1 à CCp, CD1 à CDq) destinés à être montés sur l'hydrocureur (2), ledit second ensemble (20, 21) de capteurs (CC1 à CCp, CD1 à CDq) comportant des capteurs (CC1 à CCp, CD1 à CDq) configurés pour mesurer des valeurs de paramètres liés audit hydrocureur (2) et/ou à son environnement.

6. Dispositif selon la revendication 5,
**caractérisé en ce que** ledit second ensemble (20) de capteurs comporte des capteurs (CC1 à CCp) aptes à mesurer au moins certains des paramètres suivants de l'hydrocureur (2) :
- des paramètres d'un moteur de l'hydrocureur (2) ;
- des paramètres d'un équipement de l'hydrocureur (2).

7. Dispositif selon l'une des revendications 5 et 6,
**caractérisé en ce que** ledit second ensemble (21) de capteurs comporte des capteurs (CD1 à CDq) aptes à mesurer au moins certains des paramètres suivants de l'environnement de l'hydrocureur (2) :
- la présence d'au moins un gaz ;
- la température ;
- le vent.

8. Dispositif selon l'une quelconque des revendications précédentes, **caractérisé en ce qu'**il comporte au moins un élément d'alerte (23A, 23B)

9. Dispositif selon l'une quelconque des revendications précédentes, **caractérisé en ce qu'**il comporte une unité centrale (22) pour le traitement de valeurs mesurées.

10. Système de surveillance globale,
**caractérisé en ce qu'**il comporte au moins un dispositif de surveillance sur site (1) selon l'une quelconque des revendications 1 à 9, et une unité de contrôle à distance (31) comprenant au moins une seconde unité de transmission radioélectrique (32) apte au moins à recevoir des secondes informations émises par une première unité de transmission radioélectrique (8) de ladite unité de surveillance locale (4).

11. Système selon la revendication 10,
**caractérisé en ce que** l'unité de contrôle à distance (31) comporte au moins l'un des éléments suivants :
- au moins un élément d'alerte (37A, 37B) ;
- au moins un élément d'affichage (36) ;
- au moins un élément de traitement de données (35).

12. Système selon l'une des revendications 10 et 11,
**caractérisé en ce que** l'unité de contrôle à distance (31) comporte une unité de commande (38) configurée pour générer de ordres de commande permettant de commander à distance au moins un équipement de l'hydrocureur (2), lesdits ordres de commande étant émis par ladite seconde unité de transmission radioélectrique (32) et reçus par ladite première unité de transmission radioélectrique (8).

13. Système selon l'une des revendications 10 à 12,
**caractérisé en ce que** l'unité de contrôle à distance (31) comporte une unité d'alerte (39) configurée pour générer de ordres de déclenchement d'alerte permettant de commander à distance au moins un élément d'alerte (19A, 19B, 23A, 23B) du dispositif de surveillance sur site (1), lesdits ordres de déclenchement d'alerte étant émis par ladite seconde unité de transmission radioélectrique (32) et reçus par ladite première unité de transmission radioélectrique (8).
